# EUROPÄISCHE PATENTSCHRIFT

(11) **EP 3 128 935 B1**
(45) Veröffentlichungstag und Bekanntmachung des Hinweises auf die Patenterteilung: **08.05.2019**
(21) Anmeldenummer: 15714799.2
(22) Anmeldetag: 01.04.2015
(51) Int. Cl.: A61B 17/88, B25B 15/02, B25B 23/14, B25B 23/142, A61B 90/00

(54) **WERKZEUGVORRICHTUNG, INSBESONDERE HANDWERKZEUGVORRICHTUNG, MIT DREHMOMENTBEGRENZUNG**
TOOL DEVICE, IN PARTICULAR HAND-HELD TOOL DEVICE, FEATURING TORQUE LIMITATION
DISPOSITIF D'OUTIL, EN PARTICULIER UN DISPOSITIF D'OUTIL À MAIN, À LIMITATION DE COUPLE

(30) Priorität: 09.04.2014 DE 102014105078
(43) Veröffentlichungstag der Anmeldung: 15.02.2017
(73) Patentinhaber: SMC Innovation GmbH, 6330 Cham (CH)
(72) Erfinder: Jansen-Troy, Arne, 78333 Stockach (DE)
(74) Vertreter: Daub, Thomas
(86) Internationale Anmeldenummer: PCT/EP2015/057232
(87) Internationale Veröffentlichungsnummer: WO 2015/155097

(56) Entgegenhaltungen:
- US-A1- 2008 016 990

## Beschreibung

### Stand der Technik

Die Erfindung betrifft eine Werkzeugvorrichtung, insbesondere eine Handwerkzeugvorrichtung, zur Verwendung in einem chirurgischen Verfahren nach dem Patentanspruch 1.

Es ist bereits eine Werkzeugvorrichtung zur Verwendung in einem chirurgischen Verfahren, mit einer Werkzeugaufnahme, einer Bedieneinheit und einer Drehmomentbegrenzungseinheit mit zumindest einem Drehmomentbegrenzungselement bekannt, wobei die Drehmomentbegrenzungseinheit zwischen der Bedieneinheit und der Werkzeugaufnahme angeordnet ist.

Aus der US 2008/0016990 A1 ist bereits eine Werkzeugvorrichtung gemäß dem Oberbegriff des Patentanspruchs 1 bekannt.

Im Medizinbereich, insbesondere während eines chirurgischen Verfahrens werden hohe Anforderungen an eine Präzision von Werkzeugvorrichtungen gestellt. Dies betrifft unter anderem ein maximales Drehmoment, mit dem beispielweise Schrauben eingedreht werden.

Die Aufgabe der Erfindung besteht insbesondere darin, eine gattungsgemäße Werkzeugvorrichtung mit einer erhöhten Präzision bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Werkzeugvorrichtung, insbesondere einer Handwerkzeugvorrichtung, zur Verwendung in einem chirurgischen Verfahren, mit einer Werkzeugaufnahme, einer Bedieneinheit und einer Drehmomentbegrenzungseinheit mit zumindest einem Drehmomentbegrenzungselement, wobei die Drehmomentbegrenzungseinheit zwischen der Bedieneinheit und der Werkzeugaufnahme angeordnet ist.

Erfindungsgemäß wird vorgeschlagen, dass das zumindest eine Drehmomentbegrenzungselement dazu vorgesehen ist, bei einem Überschreiten eines vorgegebenen Drehmoments zerstört zu werden. Dadurch kann eine Drehmomentübertragung bei einem Überschreiten des vorgegebenen Drehmoments auf besonders sichere Weise unterbrochen werden. Unter einer "Werkzeugaufnahme" soll in diesem Zusammenhang insbesondere ein Element der Werkzeugvorrichtung verstanden werden, das dazu vorgesehen ist, ein Einsatzwerkzeug drehfest zu halten. Bevorzugt ist die Werkzeugaufnahme dazu vorgesehen, das Einsatzwerkzeug von einem Bediener auf eine wieder lösbare Weise aufzunehmen. Unter einer "Bedieneinheit" soll in diesem Zusammenhang insbesondere eine Einheit verstanden werden, die dazu vorgesehen ist, von einem Benutzer der Werkzeugvorrichtung bedient zu werden. Die Bedieneinheit umfasst vorteilhaft zumindest einen Handgriff. Es ist in diesem Zusammenhang denkbar, dass die Bedieneinheit ein T-Griffstück aufweist. Unter einer "Drehmomentbegrenzungseinheit" soll in diesem Zusammenhang insbesondere eine Einheit verstanden werden, die zur Begrenzung eines zu übertragenden Drehmoments vorgesehen ist. Vorteilhaft entkoppelt die Drehmomentbegrenzungseinheit eine Drehmomentübertragung zwischen der Bedieneinheit und der Werkzeugaufnahme bei einem Überschreiten eines voreingestellten Drehmoments. Das zumindest eine Drehmomentbegrenzungselement kann keramisch, metallisch oder bevorzugt aus einem Kunststoff ausgebildet sein. Unter "zerstören" soll in diesem Zusammenhang insbesondere vom benachbarten Drehmomentbegrenzungselement oder von einem benachbarten Führungselement abtrennen verstanden werden. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

In einer weiteren Ausgestaltung der Werkzeugvorrichtung wird vorgeschlagen, dass die Drehmomentbegrenzungseinheit dazu vorgesehen ist, nach einer begrenzten Anzahl an Drehmomentüberschreitungen eine Drehmomentübertragung zwischen der Bedieneinheit und der Werkzeugaufnahme irreversibel zu unterbrechen. Dadurch kann eine Entsorgung der Werkzeugvorrichtung nach einer begrenzten Anzahl an Drehmomentüberschreitungen sichergestellt werden. Eine Wiederverwendung der Werkzeugvorrichtung nach der begrenzten Anzahl an Drehmomentüberschreitungen kann vorteilhaft vermieden werden. Eine Gesundheitsgefährdung durch Verwendung einer verunreinigten Werkzeugvorrichtung kann besonders vorteilhaft reduziert werden. Unter der Wendung "nach einer begrenzten Anzahl" soll in diesem Zusammenhang insbesondere nach einem Mal, vorzugsweise nach maximal fünf Mal, besonders vorteilhaft nach maximal zehn Mal verstanden werden.

Erfindungsgemäß wird vorgeschlagen, dass das zumindest eine Drehmomentbegrenzungselement auf einer Arbeitsdrehachse der Werkzeugaufnahme angeordnet ist. Dadurch kann die Werkzeugvorrichtung besonders kompakt ausgebildet werden. Unter einer "Arbeitsdrehachse" soll in diesem Zusammenhang insbesondere eine Achse verstanden werden, um welche die Werkzeugaufnahme und/oder vorteilhaft die Werkzeugvorrichtung in einem Betriebsmodus, insbesondere bei einem Ein- oder Ausdrehen einer Schraube, gedreht wird. Vorteilhaft liegt ein Schwerpunkt des zumindest einen Drehmomentbegrenzungselements auf der Arbeitsdrehachse oder in einem Abstand von weniger als 5 mm, bevorzugt von weniger als 2 mm, besonders bevorzugt von weniger als 1 mm von der Arbeitsdrehachse entfernt.

Erfindungsgemäß wird vorgeschlagen, dass die Drehmomentbegrenzungseinheit eine Mehrzahl an Drehmomentbegrenzungselementen aufweist, die dazu vorgesehen sind, bei einem Überschreiten eines vorgegebenen Drehmoments zerstört zu werden. Dadurch kann eine begrenzte Anzahl an möglichen Drehmomentüberschreitungen auf eine fehlersichere Weise festgelegt werden.

Die Werkzeugvorrichtung kann besonders kompakt ausgebildet werden, wenn die Drehmomentbegrenzungselemente einstückig miteinander ausgebildet sind. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss, einem einzelnen Rohling und/oder vorteilhaft durch eine Herstellung in einem Ein- oder Mehrkomponentenspritzverfahren.

Erfindungsgemäß umfasst die Werkzeugvorrichtung eine Drehmomentübertragungseinheit, die dazu vorgesehen ist, jeweils ein Drehmomentbegrenzungselement aufzunehmen und bei dem Überschreiten des vorgegebenen Drehmoments zu zerstören, wodurch eine konstruktiv einfache Drehmomentbegrenzung erzielt werden kann. Unter einer "Drehmomentübertragungseinheit" soll in diesem Zusammenhang insbesondere eine Einheit verstanden werden, die in zumindest einem Betriebsmodus ein Drehmoment von einer Bedieneinheit zu einer Werkzeugaufnahme überträgt. Die Drehmomentübertragungseinheit ist vorteilhaft dazu vorgesehen, bei einer Drehmomentübertragung eine Kraft auf das Drehmomentbegrenzungselement auszuüben.

Ferner wird vorgeschlagen, dass die Drehmomentübertragungseinheit dazu vorgesehen ist, jeweils ein Drehmomentbegrenzungselement durch eine Torsion zu zerstören. Dadurch kann eine besonders zuverlässige Zerstörung des Drehmomentbegrenzungselements bei einem vorbestimmten Drehmoment erzielt werden. Es ist alternativ auch denkbar, dass die Drehmomentübertragungseinheit dazu vorgesehen ist, jeweils ein Drehmomentbegrenzungselement durch Ausübung einer Scherkraft zu zerstören.

Erfindungsgemäß wird vorgeschlagen, dass die Werkzeugvorrichtung eine Magazineinheit umfasst, die dazu vorgesehen ist, eine Vortriebskraft auf die Drehmomentbegrenzungselemente auszuüben, um diese einzeln in eine Drehmomentübertragungseinheit einzuführen. Dadurch kann ein halbautomatisches oder auch ein automatisches Befüllen der Drehmomentübertragungseinheit mit dem zumindest einen Drehmomentbegrenzungselement in einem Betriebsmodus erfolgen. Bevorzugt weist die Magazineinheit zumindest eine Druckfeder auf, die zur Ausübung der Vortriebskraft auf das zumindest eine Drehmomentbegrenzungselement vorgesehen ist. Vorteilhaft ist die Druckfeder aus Nitinol ausgebildet.

Eine besonders exakte Drehmomentbegrenzung kann erreicht werden, wenn die Magazineinheit eine Ausgleichseinheit umfasst, die dazu vorgesehen ist, die Vortriebskraft für jedes der Drehmomentbegrenzungselemente zumindest im Wesentlichen konstant zu halten. Unter einer "Ausgleichseinheit" soll in diesem Zusammenhang insbesondere eine Einheit verstanden werden, die eine Federeinstelleinheit und/oder vorteilhaft eine Reibungseinstelleinheit umfasst. Unter einer "Federeinstelleinheit" soll in diesem Zusammenhang insbesondere eine Einheit verstanden werden, die dazu vorgesehen ist, eine Federkraft der Druckfeder einzustellen. Bevorzugt ist die Federeinstelleinheit dazu vorgesehen, eine Länge der Druckfeder einzustellen. Vorteilhaft weist die Federeinstelleinheit zumindest ein Gewindeelement auf, das zur Anlage an der Druckfeder vorgesehen ist. Unter einer "Reibungseinstelleinheit" soll in diesem Zusammenhang insbesondere eine Einheit verstanden werden, die dazu vorgesehen ist, eine Reibkraft zwischen dem zumindest einen Drehmomentbegrenzungselement und der Drehmomentübertragungseinheit, insbesondere in Abhängigkeit von einer Federkraft, einzustellen.

Ein Verlust von zerstörten Drehmomentbegrenzungselementen kann vermieden werden, wenn die Bedieneinheit zumindest eine Aufnahmekammer aufweist, die zur Aufnahme von zumindest einem zerstörten Drehmomentbegrenzungselement der Drehmomentbegrenzungseinheit vorgesehen ist. Bevorzugt ist die Aufnahmekammer durch zwei Bedienteile der Bedieneinheit begrenzt.

Ferner wird vorgeschlagen, dass die Bedieneinheit zumindest ein Bedienteil zur Bildung eines Aufnahmeraums umfasst, in welchem die Drehmomentbegrenzungseinheit in einem montierten Zustand angeordnet ist. Vorteilhaft ist das Bedienteil derart ausgebildet, dass ein zerstörungsfreier Zugang zum Aufnahmeraum vermieden ist. Unter einem "Zugang" soll in diesem Zusammenhang insbesondere eine freie Öffnung verstanden werden, die einen Hand-, Finger- und/oder Werkzeugeingriff in den Aufnahmeraum ermöglicht. Das Bedienteil weist vorteilhaft keinen Zugang mit einem maximalen Öffnungsdurchmesser von mehr als 3 mm auf. Bevorzugt weist die Bedieneinheit zwei Bedienteile auf. Vorteilhaft umfasst die Bedieneinheit eine Rasteinheit, die zur Verrastung der beiden Bedienteile vorgesehen ist.

Weist die Werkzeugvorrichtung einen Durchgangskanal auf, der die Werkzeugaufnahme, die Bedieneinheit und die Drehmomentbegrenzungseinheit zumindest zu einem Großteil durchdringt, kann vorteilhaft ein Kirschnerdraht und/oder ein beliebiger Führungsdraht aufgenommen werden. Vorteilhaft weist der Durchgangskanal einen Durchmesser von weniger als 3 mm, bevorzugt von weniger als 1 mm, auf. Bevorzugt durchdringt der Durchgangskanal die Werkzeugaufnahme, die Bedieneinheit, die Drehmomentbegrenzungseinheit und das zumindest eine Drehmomentbegrenzungselement vollständig. Besonders vorteilhaft weist der Durchgangskanal einen runden Querschnitt auf. Weiterhin vorteilhaft entspricht eine Symmetrieachse des Durchgangskanals der Arbeitsdrehachse der Werkzeugaufnahme.

Weiterhin wird vorgeschlagen, dass die Werkzeugvorrichtung eine Füllstandsanzeigeeinheit aufweist, die dazu vorgesehen ist, eine verbleibende Anzahl an Drehmomentbegrenzungselementen auszugeben. Unter einer "Füllstandsanzeigeeinheit" soll in diesem Zusammenhang insbesondere eine Einheit verstanden werden, die zu einer akustischen und/oder vorzugsweise optischen Ausgabe einer verbleibenden Anzahl an Drehmomentbegrenzungselementen vorgesehen ist. Zusätzlich und/oder alternativ ist die Füllstandsanzeigeeinheit zur Anzeige bereits verbrauchter Drehmomentbegrenzungselemente vorgesehen.

Es wird ferner ein Verfahren zur Begrenzung eines Drehmoments einer Werkzeugvorrichtung vorgeschlagen, die eine Werkzeugaufnahme und eine Bedieneinheit umfasst, wobei ein Bediener über die Bedieneinheit und eine Drehmomentbegrenzungseinheit ein Drehmoment auf die Werkzeugaufnahme ausübt, wobei bei einem vorgegebenen Drehmoment zumindest ein Drehmomentbegrenzungselement der Drehmomentbegrenzungseinheit zerstört wird. Dadurch kann eine Drehmomentübertragung bei einem Überschreiten des vorgegebenen Drehmoments auf besonders sichere Weise unterbrochen werden.

Des Weiteren wird vorgeschlagen, dass nach einer vorbestimmbaren Anzahl an Drehmomentüberschreitungen eine Drehmomentübertragung zwischen der Bedieneinheit und der Werkzeugaufnahme irreversibel unterbrochen wird. Dadurch kann eine Entsorgung der Werkzeugvorrichtung nach einer begrenzten Anzahl an Drehmomentüberschreitungen sichergestellt werden. Eine Wiederverwendung der Werkzeugvorrichtung nach der begrenzten Anzahl an Drehmomentüberschreitungen kann vorteilhaft vermieden werden. Eine Gesundheitsgefährdung durch Verwendung einer verunreinigten Werkzeugvorrichtung kann somit besonders vorteilhaft reduziert werden.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine Seitenansicht einer Werkzeugvorrichtung,
- Fig. 2: eine Seitenansicht der Werkzeugvorrichtung mit nur einem Bedienteil und
- Fig. 3: einen perspektivischen Teilschnitt durch die Werkzeugvorrichtung.

### Beschreibung des Ausführungsbeispiels

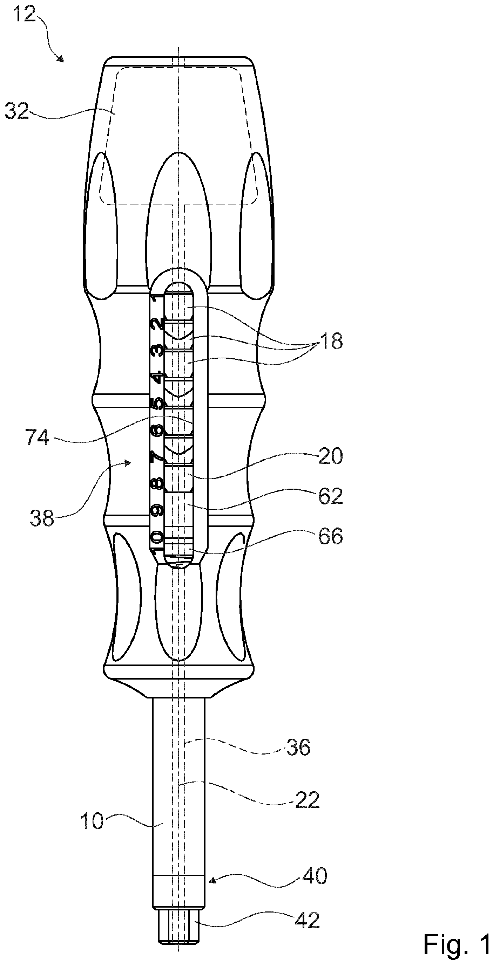

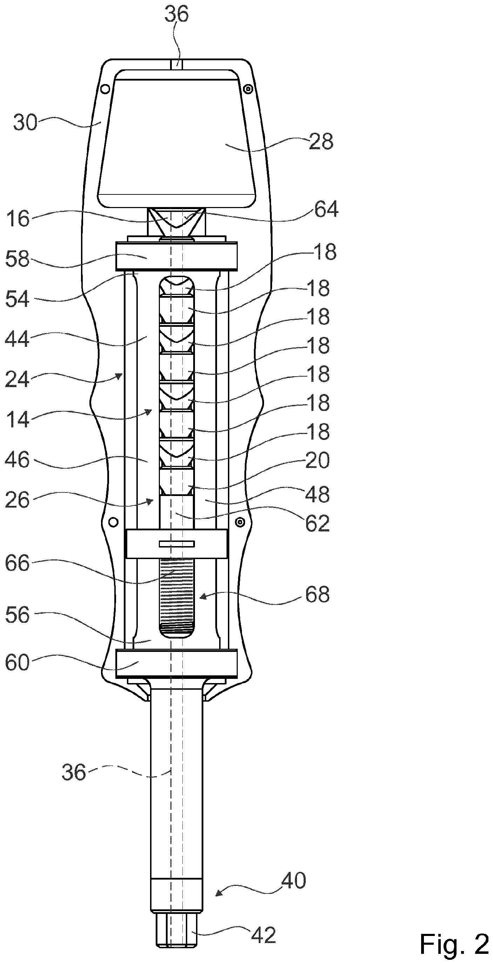

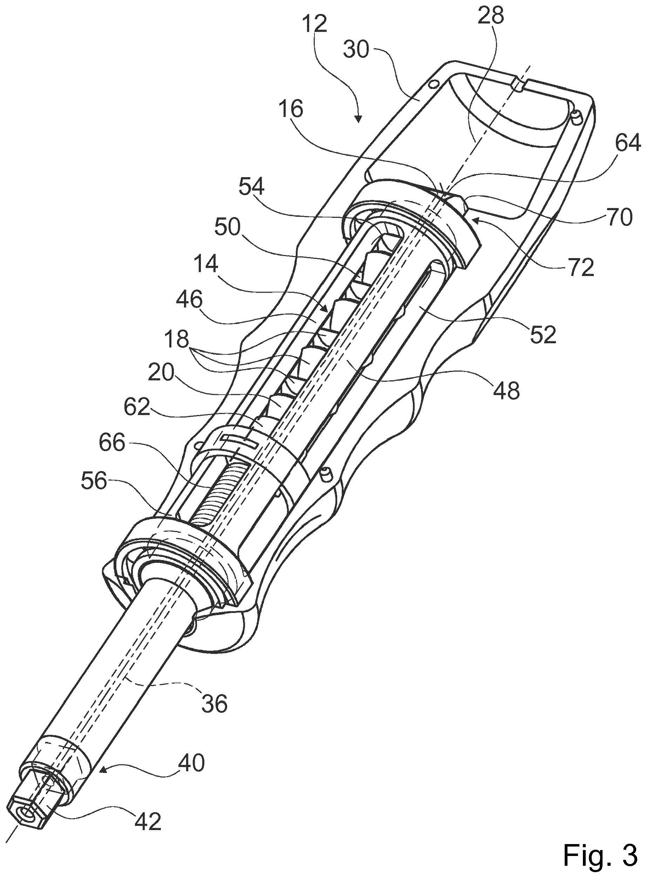

Die Figuren 1 bis 3 zeigen eine Werkzeugvorrichtung, die als Handwerkzeugvorrichtung ausgebildet ist. Die Werkzeugvorrichtung ist zur Verwendung in einem chirurgischen Verfahren vorgesehen. Die Werkzeugvorrichtung weist eine Werkzeugaufnahme 10 auf. Die Werkzeugaufnahme 10 ist an einem freien Ende 40 mit einem Einsatzwerkzeug 42 lösbar gekoppelt. Die Werkzeugaufnahme 10 ist dazu vorgesehen, das Einsatzwerkzeug 42 drehfest zu halten. Das Einsatzwerkzeug 42 ist dabei von einem Bediener auswechselbar. Das Einsatzwerkzeug 42 ist als Sechskantbit ausgebildet. Somit ist das Einsatzwerkzeug 42 in diesem Fall formkomplementär zu einem Innensechskant einer nicht dargestellten Schraube. Grundsätzlich ist es auch denkbar, dass die Werkzeugaufnahme 10 selbst dazu vorgesehen ist, mit einer Schraube drehfest gekoppelt zu werden. Bei einem Bearbeitungsvorgang dreht sich die Werkzeugvorrichtung um eine Arbeitsdrehachse 22 der Werkzeugaufnahme 10.

Die Werkzeugvorrichtung umfasst ferner eine Magazineinheit 24. Die Magazineinheit 24 umfasst einen Aufnahmerahmen 44. Der Aufnahmerahmen 44 ist zur Aufnahme einer Drehmomentbegrenzungseinheit 14 der Werkzeugvorrichtung vorgesehen. Der Aufnahmerahmen 44 umfasst vier Haltestäbe 46, 48, 50, 52. Die Haltestäbe 46, 48, 50, 52 erstrecken sich in einer Haupterstreckungsrichtung parallel zur Arbeitsdrehachse 22 der Werkzeugaufnahme 10. Die Haltestäbe 46, 48, 50, 52 sind an freien Längsenden miteinander verbunden. Die Magazineinheit 24 weist hierfür ein oberes Abschlussteil 54 und ein unteres Abschlussteil 56 auf. Das obere Abschlussteil 54 und das untere Abschlussteil 56 sind zum Verbinden der Haltestäbe 46, 48, 50, 52 vorgesehen. Das obere Abschlussteil 54 weist eine kreisrunde Außenkontur auf. Das untere Abschlussteil 56 weist eine kreisrunde Außenkontur auf. Das untere Abschlussteil 56 ist näher an der Werkzeugaufnahme 10 angeordnet, als das obere Abschlussteil 54. Die Werkzeugvorrichtung weist ferner zwei Kugellager 58, 60 auf, welche die beiden Abschlussteile 54, 56 umgreifen. Es ist in diesem Zusammenhang ebenfalls denkbar, dass die Magazineinheit 24 einfache Gleitlager anstelle der Kugellager 58, 60 umfasst.

Innerhalb der Magazineinheit 24 ist die Drehmomentbegrenzungseinheit 14 angeordnet. Die Drehmomentbegrenzungseinheit 14 weist zehn Drehmomentbegrenzungselemente 16, 18, 20 auf. Die Drehmomentbegrenzungselemente 16, 18, 20 sind entlang der Arbeitsdrehachse 22 hintereinander angeordnet. Die Drehmomentbegrenzungseinheit 14 weist ferner ein Führungselement 62 auf, das an einem werkzeugaufnahmezugewandten Ende der Drehmomentbegrenzungseinheit 14 angeordnet ist. Das Führungselement 62 ist zylinderförmig ausgebildet. Das Führungselement 62 weist vier Längsnuten auf, in welche die Haltestäbe 46, 48, 50, 52 eingreifen. Die Haltestäbe 46, 48, 50, 52 bilden eine lineare Führung des Führungselements 62. Das Führungselement 62 ist drehfest relativ zu den Haltestäben 46, 48, 50, 52 gelagert.

Alle Drehmomentbegrenzungselemente 16, 18, 20 weisen eine gleiche Form auf. Jedes Drehmomentbegrenzungselement 16, 18, 20 ist zu jedem benachbarten Drehmomentbegrenzungselement 16, 18, 20 um 90° verdreht angeordnet. Im Folgenden wird beispielhaft die Form des obersten Drehmomentbegrenzungselements 16 beschrieben. Das Drehmomentbegrenzungselement 16 ist als Stumpfkegel ausgebildet, wobei zwei voneinander abgewandte ebene Drehmomentübertragungsflächen 64, die senkrecht zu einer Grundfläche und einer Deckfläche des Stumpfkegels verlaufen, in eine Mantelfläche eingebracht sind. Die Drehmomentübertragungsflächen 64 verlaufen parallel zueinander. Die Drehmomentübertragungsflächen 64 liegen an den Haltestäben 46, 48, 50, 52 an. Die Haltestäbe 46, 48, 50, 52 bilden eine lineare Führung der Drehmomentbegrenzungseinheit 14. Die Drehmomentbegrenzungseinheit 14 ist drehfest relativ zu den Haltestäben 46, 48, 50, 52 gelagert.

Die Drehmomentbegrenzungselemente 16, 18, 20 sind einstückig miteinander ausgebildet. Genauer gesagt sind die Drehmomentbegrenzungselemente 16, 18, 20 in einem Spritzgussverfahren hergestellt. Die Drehmomentbegrenzungselemente 16, 18, 20 sind aus einem Kunststoff ausgebildet. Ein Fachmann wird hierbei einen ihm als sinnvoll erscheinenden Kunststoff verwenden, wie insbesondere Polyethylen, Polypropylen, Polystyrol, Polyester, Polyvinylchlorid, Polyamid, Polyethylenterephthalat, Polyurethan oder Phenoplast. Das Führungselement 62 ist an einer Deckfläche eines untersten Drehmomentbegrenzungselements 20 angeordnet. Genauer gesagt ist das Führungselement 62 mit dem untersten Drehmomentbegrenzungselement 20 einstückig ausgebildet. Das unterste Drehmomentbegrenzungselement 20 liegt der Werkzeugaufnahme 10 in einem Betriebsmodus am nächsten. Die Drehmomentbegrenzungselemente 16, 18, 20 sind auf der Arbeitsdrehachse 22 der Werkzeugaufnahme 10 angeordnet. Die Arbeitsdrehachse 22 entspricht dabei einer Rotationssymmetrieachse, zu welcher die Drehmomentbegrenzungselemente 16, 18, 20 rotationssymmetrisch ausgebildet sind.

Die Magazineinheit 24 weist ferner eine Druckfeder 66 auf. Die Druckfeder 66 ist als Schraubendruckfeder ausgebildet. Die Druckfeder 66 besteht aus Nitinol. Es ist in diesem Zusammenhang auch denkbar, dass die Druckfeder 66 aus einem anderen, dem Fachmann als sinnvoll erscheinenden Material, wie insbesondere einem Kunststoff, einer Kupfer-Beryllium-Legierung, einem Gummi, einem Faserverbundwerkstoff oder bevorzugt aus Edelstahl besteht. Die Druckfeder 66 ist in einem der Werkzeugaufnahme 10 zugewandten Ende 68 der Magazineinheit 24 angeordnet. Die Druckfeder 66 stützt sich an einem Ende auf dem unteren Abschlussteil 56 ab. An einem anderen Ende stützt sich die Druckfeder 66 gegen das Führungselement 62 ab. Das Führungselement 62 drückt folglich gegen die Drehmomentbegrenzungselemente 16, 18, 20. Die Druckfeder 66 übt somit eine Vortriebskraft auf die Drehmomentbegrenzungseinheit 14 aus.

Die Werkzeugvorrichtung weist ferner eine Bedieneinheit 12 auf. Die Bedieneinheit 12 umfasst zwei Bedienteile 30, 32. Die Bedienteile 30, 32 bilden einen Aufnahmeraum 34, in welchem die Drehmomentbegrenzungseinheit 14 in einem montierten Zustand angeordnet ist. Dabei sind die Bedienteile 30, 32 derart ausgebildet, dass ein zerstörungsfreier Zugang zum Aufnahmeraum 34 vermieden ist.

Die Bedienteile 30, 32 sind als Griffschalenteile ausgebildet. Die Bedienteile 30, 32 sind zumindest im Wesentlichen symmetrisch zueinander ausgebildet. Die Bedienteile 30, 32 sind zusammengefügt. Im gezeigten Ausführungsbeispiel sind die Bedienteile 30, 32 stoffschlüssig miteinander verbunden. Die stoffschlüssige Verbindung ist durch eine Klebeverbindung hergestellt. Es ist in diesem Zusammenhang auch denkbar, die Bedienteile 30, 32 durch eine kraftschlüssige und/oder formschlüssige Verbindung miteinander zu koppeln. Dabei wird ein Fachmann insbesondere eine Rastverbindung zwischen den Bedienteilen 30, 32 in Betracht ziehen. Die Bedienteile 30, 32 sind untrennbar miteinander verbunden. Ein Trennen der Bedienteile 30, 32 ist also nicht zerstörungsfrei möglich. Die zusammengefügten Bedienteile 30, 32 bilden einen Griff aus. Die Bedienteile 30, 32 sind drehfest mit Außenringen der Kugellager 58, 60 verbunden. Die Magazineinheit 24 ist drehfest mit Innenringen der Kugellager 58, 60 verbunden.

Die Werkzeugvorrichtung umfasst ferner eine Drehmomentübertragungseinheit 72. Die Drehmomentübertragungseinheit 72 ist dazu vorgesehen, jeweils eines der Drehmomentbegrenzungselemente 16, 18, 20 aufzunehmen. Die Drehmomentübertragungseinheit 72 umfasst eine Drehmomentbegrenzungselementaufnahme 70. Die Drehmomentbegrenzungselementaufnahme 70 bildet eine Ausnehmung innerhalb der Bedieneinheit 12 aus. Die Drehmomentbegrenzungselementaufnahme 70 ist in einer senkrecht zur Arbeitsdrehachse 22 verlaufenden Ebene formkomplementär zu den Drehmomentbegrenzungselementen 16, 18, 20. Ein Drehmomentbegrenzungselement 16, 18, 20, welches innerhalb der Drehmomentbegrenzungselementaufnahme 70 angeordnet ist, ist somit drehfest mit der Bedieneinheit 12 verbunden.

Bei einer Verwendung der Werkzeugvorrichtung wird von Hand ein Drehmoment auf die Bedieneinheit 12 gegeben. Die Werkzeugaufnahme 10 ist über die Drehmomentbegrenzungseinheit 14 drehfest mit der Bedieneinheit 12 gekoppelt. Die Werkzeugaufnahme 10 wirkt gegen das erzeugte Drehmoment des Bedienteils 12. Auf das in der Drehmomentbegrenzungselementaufnahme 70 angeordnete Drehmomentbegrenzungselement 16 wirkt somit zum benachbarten Drehmomentbegrenzungselement 18 eine Torsion. Die Drehmomentbegrenzungseinheit 14 ist zwischen der Bedieneinheit 12 und der Werkzeugaufnahme 10 angeordnet. Die Drehmomentübertragungseinheit 72 ist dazu vorgesehen, bei einer Drehmomentübertragung von der Bedieneinheit 12 zur Werkzeugaufnahme 10 eine Kraft auf das Drehmomentbegrenzungselement 16 auszuüben.

Das in der Drehmomentübertragungseinheit 72 angeordnete Drehmomentbegrenzungselement 16 ist dazu vorgesehen, bei einem Überschreiten eines vorgegebenen Drehmoments zerstört zu werden. Anders ausgedrückt zerstört die Drehmomentübertragungseinheit 72 bei dem Überschreiten des vorgegebenen Drehmoments das aufgenommene Drehmomentbegrenzungselement 16. Dabei wird das aufgenommene Drehmomentbegrenzungselement 16 vom benachbarten Drehmomentbegrenzungselement 18 abgetrennt. Das Abtrennen erfolgt durch die Torsion zwischen dem aufgenommenen Drehmomentbegrenzungselement 16 und dem benachbarten Drehmomentbegrenzungselement 18. Die Bedieneinheit 12 dreht sich danach etwa eine Viertelumdrehung frei um die Werkzeugaufnahme 10.

Die Bedieneinheit 12 umfasst ferner eine Aufnahmekammer 28. Die Aufnahmekammer 28 ist zur Aufnahme von zerstörten Drehmomentbegrenzungselementen 16, 18, 20 der Drehmomentbegrenzungseinheit 14 vorgesehen. Die Aufnahmekammer 28 ist durch die zwei Bedienteile 30, 32 der Bedieneinheit 12 begrenzt. Das abgetrennte und somit zerstörte Drehmomentbegrenzungselement 16, 18, 20 wird durch eine Schwerkraft und/oder durch die Vortriebskraft in die Aufnahmekammer 28 bewegt. Durch die Viertelumdrehung der Bedieneinheit 12 um die Werkzeugaufnahme 10 greift das vormals benachbarte Drehmomentbegrenzungselement 18 in die Drehmomentbegrenzungselementaufnahme 70 ein. Die Vortriebskraft der Druckfeder 66 führt das vormals benachbarte Drehmomentbegrenzungselement 18 somit in die Drehmomentübertragungseinheit 72 ein. Somit ist die Bedieneinheit 12 gegenüber der Werkzeugaufnahme 10 bis zum einem Erreichen des vorgegebenen Drehmoments wieder drehfest gekoppelt. Dieser Ablauf erfolgt bis zum Abtrennen des untersten Drehmomentbegrenzungselements 20 von dem Führungselement 62. Anschließend ist die Werkzeugvorrichtung unbrauchbar und muss entsorgt werden. Alle abgetrennten Drehmomentbegrenzungselemente 16, 18, 20 sind dann lose innerhalb der Aufnahmekammer 28 angeordnet. Die Drehmomentbegrenzungseinheit 14 ist somit dazu vorgesehen, nach einer begrenzten Anzahl an Drehmomentüberschreitungen eine Drehmomentübertragung zwischen der Bedieneinheit 12 und der Werkzeugaufnahme 10 irreversibel zu unterbrechen.

Wie zuvor erwähnt, bewegt die Druckfeder 66 nach jeder Drehmomentüberschreitung ein weiteres Drehmomentbegrenzungselement 18, 20 in die Drehmomentübertragungseinheit 72, bis das unterste Drehmomentbegrenzungselement 20 vom Führungselement 62 abgetrennt ist. Dabei entspannt sich die Druckfeder 66. Folglich nimmt die Federkraft der Druckfeder 66 ab. Entgegen der Federkraft wirkt eine Reibungskraft zwischen der Drehmomentbegrenzungseinheit 14 und der Magazineinheit 24. Um die Vortriebskraft trotz einer Entspannung der Druckfeder 66 zumindest im Wesentlichen konstant zu halten, umfasst die Magazineinheit 24 eine Ausgleichseinheit 26. Die Ausgleichseinheit 26 ist dazu vorgesehen, die Vortriebskraft für jedes Drehmomentbegrenzungselement 16, 18, 20 zumindest im Wesentlichen konstant zu halten. Die Ausgleichseinheit 26 umfasst eine Reibungseinstelleinheit. Die Reibungseinstelleinheit umfasst vier voneinander divergierende Flächen, die von den Haltestäben 46, 48, 50, 52 ausgebildet sind. Die Flächen entfernen sich von der Werkzeugaufnahme 10 in Richtung der Drehmomentübertragungseinheit 14 um etwa 0,1 mm. Ein Fachmann wird in diesem Zusammenhang eine, ihm sinnvoll erscheinende Divergenz festlegen, um eine konstante Vortriebskraft zu erzielen. Es ist in diesem Zusammenhang auch denkbar, dass der Fachmann eine Federeinstelleinheit vorsieht, die dazu vorgesehen ist, die Federkraft der Druckfeder 66 einzustellen.

Die Werkzeugvorrichtung weist einen Durchgangskanal 36 auf. Der Durchgangskanal 36 durchdringt die Werkzeugaufnahme 10, die Bedieneinheit 12 und die Drehmomentbegrenzungseinheit 14 vollständig. Der Durchgangskanal 36 ist zylinderförmig ausgebildet. Eine Symmetrieachse des Durchgangskanals 36 liegt auf der Arbeitsdrehachse 22. Der Durchgangskanal 36 weist einen geringeren Durchmesser auf als die Drehmomentbegrenzungseinheit 14. Der Durchgangskanal 36 ist frei von Krümmungen. Eine Durchführung der Drehmomentbegrenzungseinheit 14 durch den Durchgangskanal 36 ist somit vermieden. Der Durchgangskanal 36 ist zur Führung eines Kirschnerdrahts vorgesehen. Der Durchgangskanal 36 weist hierbei einen Durchmesser von weniger als 1 mm auf.

Die Werkzeugvorrichtung weist ferner eine Füllstandsanzeigeeinheit 38 auf. Die Füllstandsanzeigeeinheit 38 ist dazu vorgesehen, eine verbleibende Anzahl an Drehmomentbegrenzungselementen 16, 18, 20 auszugeben. Die Füllstandsanzeigeeinheit 38 weist hierzu eine Anzeigeausnehmung 74 aus. Die Anzeigeausnehmung 74 verläuft schlitzartig in einem Bedienteil 32 der Bedienteile 30, 32. Alternativ verläuft die Anzeigeausnehmung 74 in beiden Bedienteilen 30, 32. Die Anzeigeausnehmung 38 verläuft parallel zur Arbeitsdrehachse 22. Die Anzeigeausnehmung 38 ist zur optischen Überprüfung eines Füllstands der Magazineinheit 24 vorgesehen. Eine Breite der Anzeigeausnehmung 74 beträgt weniger als 3 mm. Eine Durchführung der Drehmomentbegrenzungseinheit 14 durch die Anzeigeausnehmung 74 ist vermieden. Die Füllstandsanzeigeeinheit 38 umfasst ferner eine Nummerierung. Die Nummerierung umfasst die Zahlen 1 bis 10. Die Zahlen sind den einzelnen Drehmomentbegrenzungselementen 16, 18, 20 zugeordnet.

## Patentansprüche

1. Werkzeugvorrichtung, insbesondere Handwerkzeugvorrichtung, zur Verwendung in einem chirurgischen Verfahren, mit einer Werkzeugaufnahme (10), einer Bedieneinheit (12), einer Drehmomentbegrenzungseinheit (14), einer Drehmomentübertragungseinheit (72) und einer Magazineinheit (24), wobei die Drehmomentbegrenzungseinheit (14) zwischen der Bedieneinheit (12) und der Werkzeugaufnahme (10) angeordnet ist und eine Mehrzahl an Drehmomentbegrenzungselementen (16, 18, 20) aufweist, die dazu vorgesehen sind, bei einem Überschreiten eines vorgegebenen Drehmoments zerstört zu werden, wobei die Magazineinheit (24) dazu vorgesehen ist, eine Vortriebskraft auf die Drehmomentbegrenzungselemente (16, 18, 20) auszuüben, um diese einzeln in die Drehmomentübertragungseinheit (72) einzuführen, und dass die Drehmomentübertragungseinheit (72) dazu vorgesehen ist, jeweils ein Drehmomentbegrenzungselement (16, 18, 20) aufzunehmen und bei dem Überschreiten des vorgegebenen Drehmoments zu zerstören, **dadurch gekennzeichnet, dass** die Drehmomentbegrenzungselemente (16, 18, 20) auf einer Arbeitsdrehachse (22) der Werkzeugaufnahme (10) angeordnet sind .

2. Werkzeugvorrichtung nach Anspruch 1, **dadurch gekennzeichnet, dass** die Drehmomentbegrenzungseinheit (14) dazu vorgesehen ist, nach einer begrenzten Anzahl an Drehmomentüberschreitungen eine Drehmomentübertragung zwischen der Bedieneinheit (12) und der Werkzeugaufnahme (10) irreversibel zu unterbrechen.

3. Werkzeugvorrichtung nach Anspruch 1 oder 2, **dadurch gekennzeichnet, dass** die Drehmomentbegrenzungselemente (16, 18, 20) einstückig miteinander ausgebildet sind.

4. Werkzeugvorrichtung nacheinem der vorhergehenden Ansprüche, **dadurch gekennzeichnet, dass** die Drehmomentübertragungseinheit (72) dazu vorgesehen ist, jeweils ein Drehmomentbegrenzungselement (16, 18, 20) durch eine Torsion zu zerstören.

5. Werkzeugvorrichtung nacheinem der vorhergehenden Ansprüche, **dadurch gekennzeichnet, dass** die Magazineinheit (24) eine Ausgleichseinheit (26) umfasst, die dazu vorgesehen ist, die Vortriebskraft für jedes der Drehmomentbegrenzungselemente (16, 18, 20) zumindest im Wesentlichen konstant zu halten.

6. Werkzeugvorrichtung nach einem der vorhergehenden Ansprüche, **dadurch gekennzeichnet, dass** die Bedieneinheit (12) zumindest eine Aufnahmekammer (28) aufweist, die zur Aufnahme von zumindest einem zerstörten Drehmomentbegrenzungselement (16, 18, 20) der Drehmomentbegrenzungseinheit (14) vorgesehen ist.

7. Werkzeugvorrichtung nach einem der vorhergehenden Ansprüche, **dadurch gekennzeichnet, dass** die Bedieneinheit (12) zumindest ein Bedienteil (30, 32) zur Bildung eines Aufnahmeraums (34) umfasst, in welchem die Drehmomentbegrenzungseinheit (14) in einem montierten Zustand angeordnet ist.

8. Werkzeugvorrichtung nach einem der vorhergehenden Ansprüche, **gekennzeichnet durch** einen Durchgangskanal (36), der die Werkzeugaufnahme (10), die Bedieneinheit (12) und die Drehmomentbegrenzungseinheit (14) zumindest zu einem Großteil durchdringt.

9. Werkzeugvorrichtung nacheinem der vorhergehenden Ansprüche, **gekennzeichnet durch** eine Füllstandsanzeigeeinheit (38), die dazu vorgesehen ist, eine verbleibende Anzahl an Drehmomentbegrenzungselementen (16, 18, 20) auszugeben.

## Claims

1. Tool device, in particular hand-held tool device, for use in a surgical method, comprising a tool receptacle (10), an operating unit (12), a torque limitation unit (14), a torque transmission unit (72) and a magazine unit (24), the torque limitation unit (14) being arranged between the operating unit (12) and the tool receptacle (10) and comprising a plurality of torque limitation elements (16, 18, 20) which are configured to be destroyed when a given torque is exceeded,
the magazine unit (24) being configured to exert a propulsion force onto the torque limitation elements (16, 18, 20) for the purpose of introducing them individually into the torque transmission unit (72), and
the torque transmission unit (72) being configured to accommodate respectively one torque limitation element (16, 18, 20) and to destroy said torque limitation element (16, 18, 20) when the given torque is exceeded,
**characterised in that** the torque limitation elements (16, 18, 20) are arranged on a rotary working axis (22) of the tool receptacle (10).

2. Tool device according to claim 1, **characterised in that** the torque limitation unit (14) is configured to irreversibly interrupt a torque transmission between the operating unit (12) and the tool receptacle (10) after a limited number of torque excess events.

3. Tool device according to claim 1 or 2, **characterised in that** the torque limitation elements (16, 18, 20) are embodied integrally with each other.

4. Tool device according to one of the preceding claims, **characterised in that** the torque transmission unit (72) is configured to destroy respectively one torque limitation element (16, 18, 20) by a torsion.

5. Tool device according to one of the preceding claims, **characterised in that** the magazine unit (24) comprises a compensation unit (26) that is configured to keep the propulsion force at least substantially constant for each of the torque limitation elements (16, 18, 20).

6. Tool device according to one of the preceding claims, **characterised in that** the operating unit (12) comprises at least one receiving chamber (28) that is configured to accommodate at least one destroyed torque limitation element (16, 18, 20) of the torque limitation unit (14).

7. Tool device according to one of the preceding claims, **characterised in that** the operating unit (12) comprises at least one operating part (30, 32) for forming an accommodation space (34) in which the torque limitation unit (14) is arranged in an assembled state.

8. Tool device according to one of the preceding claims, **characterised by** a pass-through channel (36) penetrating the tool receptacle (10), the operating unit (12) and the torque limitation unit (14) at least to a large extent.

9. Tool device according to one of the preceding claims, **characterised by** a filling level indication unit (38) that is configured to indicate a remaining number of torque limitation elements (16, 18, 20).

## Revendications

1. Dispositif d'outil, notamment dispositif d'outil à main, pour usage en procédé chirurgical, avec un réceptacle d'outil (10), une unité de maniement (12), une unité à limitation de couple (14), une unité à transfert de couple (72) et une unité magasin (24),
où l'unité à limitation de couple (14) est disposé entre l'unité de maniement (12) et le réceptacle d'outil (10) et comporte une pluralité d'éléments à limitation de couple (16, 18, 20) prévus à être détruits au cas où un couple donné est dépassé, l'unité magasin (24) étant configurée à exercer une force propulsive sur les éléments à limitation de couple (16, 18, 20) pour les introduire individuellement dans l'unité à transfert de couple (72), et
l'unité à transfert de couple (72) étant configurée à recevoir respectivement un élément à limitation de couple (16, 18, 20) et le détruire en dépassement du couple donné,
**caractérisé en ce que** les éléments à limitation de couple (16, 18, 20) sont disposés sur un axe rotationnel opératif (22) du réceptacle d'outil (10).

2. Dispositif d'outil selon la revendication 1,
**caractérisé en ce que** l'unité à limitation de couple (14) est configurée pour interrompre irréversiblement un transfert de couple entre l'unité de maniement (12) et le réceptacle d'outil (10) après un nombre limité des dépassements de couple.

3. Dispositif d'outil selon l'une quelconque des revendications 1 ou 2,
**caractérisé en ce que** les éléments à limitation de couple (16, 18, 20) sont réalisés intégralement l'un avec l'autre.

4. Dispositif d'outil selon l'une quelconque des revendications précédentes, **caractérisé en ce que** l'unité à transfert de couple (72) est configurée pour détruire par torsion respectivement un élément à limitation de couple (16, 18, 20).

5. Dispositif d'outil selon l'une quelconque des revendications précédentes, **caractérisé en ce que** l'unité magasin (24) comporte une unité de compensation (26), qui est configurée à maintenir la force propulsive au moins sensiblement constant pour chacun des éléments à limitation de couple (16, 18, 20).

6. Dispositif d'outil selon l'une quelconque des revendications précédentes, **caractérisé en ce que** l'unité de maniement (12) comporte au moins une chambre d'accommodation (28) prévue pour recevoir au moins un élément à limitation de couple détruit (16, 18, 20) de l'unité à limitation de couple (14).

7. Dispositif d'outil selon l'une quelconque des revendications précédentes, **caractérisé en ce que** l'unité de maniement (12) comporte au moins un élément de maniement (30, 32) pour réaliser un espace d'accommodation (34), dans lequel l'unité à limitation de couple (14) est disposé en état monté.

8. Dispositif d'outil selon l'une quelconque des revendications précédentes, **caractérisé par** un canal de passage (36) pénétrant l'accommodation d'outil (10), l'unité de maniement (12) et l'unité à limitation de couple (14) au moins en grande partie.

9. Dispositif d'outil selon l'une quelconque des revendications précédentes, **caractérisé par** une unité d'affichage de niveau de remplissage (38), qui est configurée à afficher un nombre restant d'éléments à limitation de couple (16, 18, 20).
